Europäisches Patentamt

European Patent Office

Office européen des brevets

(11) Publication number: **0 330 453**
A1

# EUROPEAN PATENT APPLICATION

(21) Application number: **89301734.3**

(22) Date of filing: **22.02.89**

(51) Int. Cl.⁴: **A 61 K 7/06**
A 61 K 9/50, B 01 J 13/00

(30) Priority: **26.02.88 US 160697**

(43) Date of publication of application:
**30.08.89 Bulletin 89/35**

(84) Designated Contracting States:
**CH DE FR GB IT LI SE**

(71) Applicant: **MINNESOTA MINING AND MANUFACTURING COMPANY**
**3M Center, P.O. Box 33427**
**St. Paul, Minnesota 55133-3427 (US)**

(72) Inventor: **Litman, Mark, A. c/o Minnesota Mining and Manufacturing Company 2501 Hudson Road Box 33427**
**St. Paul Minnesota 55133-3427 (US)**

(74) Representative: **Baillie, Iain Cameron et al**
**c/o Ladas & Parry Isartorplatz 5**
**D-8000 München 2 (DE)**

(54) Scalp treatment composition.

(57) The use of hair-loss preventing compounds with mild abrasives, especially frangible microcapsules, is believed to increase the effectiveness of the compounds.

# Description

## SCALP TREATMENT COMPOSITION

### BACKGROUND OF THE INVENTION

#### 1. Field of the Invention

The present invention relates to hair care compositions or lotions containing liquids or oils and especially cosmetic emollient oils in microcapsules for use in the application of hair restorative or baldness impeding materials to the hair and scalp.

#### 2. Background of the Art

It is fairly common to find encapsulated liquid materials in the marketplace. Technology has been available for many years to effectively provide microcapsules with liquid oleophilic ingredients. Representative processes are shown in U.S. Patents 3,016,308 and 3,516,941. These patents disclose in situ polymerization reactions in which a hydrophobic oil phase is dispersed in an aqueous phase containing resin precursors, particularly aminoplast resin precursors (to form urea/aldehyde resins and the like). High shear agitation is used to keep the capsule size small. Addition of an acid catalyst initiates the polycondensation of the aminoplast precursors, resulting in the deposition of the aminoplast resin about the dispersed droplets of the oil phase. This produces the microcapsules.

Other polycondensation encapsulation techniques are shown in U.S. Patents 3,429,827 and 4,000,087. These particular techniques are more limited in the classes of hydrophobic inner phases acceptable in the microcapsules because of reaction with the oil soluble monomer or poor solubility of the monomer in the desired hydrophobic phase.

U.S. Patent 3,930,101 teaches that, to be retained in the hydrophobic phase during high shear dispersion of a fluid particulate dispersion, it is necessary that the particulate be preferentially wetted by the hydrophobic phase. It is suggested to use suitable surfactants which adsorb to the particulate surface as a way to achieve the desired preferential wetting. It has, however, been recognized that, in the in situ polymerization of aminoplast resins method for encapsulation, the presence of surfactants interferes with the deposition of the aminoplast resin at the hydrophobic phase/water phase interface, giving poorly formed or leaky capsules. Similarly, oil soluble suspending agents could alter the wetting of many particulates. Since many of these materials contain carboxylate groups, exposure to highly acidic medias often converts them to carboxylic acid groups altering their adsorbability to the particulates.

U.S. Patent 4,450,221 teaches magnetic toners comprising lyophilic magnetic particles and a resin surrounded by a resin wall to form microcapsules. Colorants such as pigments or dyes may be included in the wall forming resin or the toner. The magnetic particles are rendered lyophilic by treatment with a titanate or silane coupling agent. The coupling agent is said to uniformly disperse the particles in the binder resin and firmly bond the magnetic particle to the resin.

Skin cleansing soaps are known to include abrasive materials such as pumice to assist in deep cleansing or abrasive removal of detritus.

### BRIEF DESCRIPTION OF THE INVENTION

Microcapsules containing colutions, dispersions or emulsions of 6-piperidino-2,4-diaminopyrimidine-3-oxide ($C_8H_{15}N_3O$, Chem. Abst. Ref. No. 38034-91-5 also known as 2,4-pyrimidine dimine, 6-(1-piperidinyl)-3-oxide) have been found to provide good hair and scalp treatments. The compound (commercially available by perscription under the name Minoxidil®) recently has been found to provide hair retainment or partial hair regrowth properties. The use of capsules with that compound therein provides an additional benefit in mild abrasive removal of dead skin or scale from the scalp and stimulation of the scalp to assist in the activity of the retainer/restorative compound. The use of emolient oils and thickeners (especially polymeric thickeners) in combination with the microcapsules can also provide a more persistent effect.

### DETAILED DESCRIPTION OF THE INVENTION

The ability of Minoxidil® (6-piperidino-2,4-diaminopyrimidine-3-oxide) to prevent progressive hair loss has been widely reported. One significant limitation in its performance is the need for daily applications of a significant quantity of the compound in order to be effective. The present cost of yearly treatment with this material is about two thousand dollars. The use of smaller amounts or less frequent applications has been found to reduce the effectiveness of the treatment and is therefore not cost effective.

It has been proposed in the present invention to apply 6-piperidino-2,4-diaminopyrimidine-3-oxide in compositions in the presence of mildly abrasive materials, particularly frangible microcapsules to provide increased efficacy to the compound. The mild abrasive removes dead skin and scale from the scalp and gently stimulates the scalp which can enable better contact and penetration of the compound into the scalp and the follicles. The use of frangible microcapsules which are capable of providing mild abrasive activity when the microcapsule shells are broken are particularly advantageous. These shells continue to break upon scrubbing (e.g., as when deep lathering with a shampoo or massaging with a lotion) so that the abrasive action diminishes and the particles are easily washed away and do not contribute to buildup on the scalp which would reduce their benefit. The presence of emolient oils and thickeners can also assist in maintaining the compound in contact with the scalp.

Liquids, oils and especially cosmetic emollient oils generally can be encapsulated by conventional procedures such as shown in U.S. Patent 3,516,941. However, even with careful control of the shear forces in the reaction vessel, the capsules tend to be too small for many commercial applications, particularly in cosmetic applications. The capsules are too difficult to rupture and the broken capsule particles are too small to provide any mildly abrasive benefits.

It has been found that the addition of soluble polymeric materials to the oils containing 6-piperidino-2,4-diaminopyrimidine-3-oxide dissolved therein enables them to form larger capsules without destroying the properties of the oils. In fact, the polymer also tends to aid the oil in adhering to the surface of skin and penetrating at a more controlled rate over a longer period of time.

It has been found that larger capsules containing cosmetic ingredients can be dispersed in skin care composition and lotion carrying media and provide additional activity including mild and controlled cleaning abrasion.

In accordance with the present invention, microcapsules are prepared by polymerization such as in situ aminoplast polymerization. The techniques disclosed, generally referred to as an in situ polymerization reaction, yield, for example, an aminoplast resin capsule wall material. In the process, a liquid or oil such as a cosmetic emollient oil phase with a polymeric material and the hair loss stabilization compound dissolved therein is dispersed in an aqueous phase containing the aminoplast resin precursors by applying shear agitation. Addition of an acid catalyst initiates the polycondensation of the aminoplast precursors, resulting in the deposition of the aminoplast resin about the dispersed droplets of the oil phase, producing the microcapsules. The oil may contain an effective amount of the 6-piperidino-2,4-diaminopyrimidine-3-oxide (e.g., up to or in excess of the solubility limit of the oil).

Typical cosmetic emollient oils are organic liquids with viscosities between 2 and 150 cp at 20°C, preferably between 2 and 100 cp. The oils preferably have molecular weights in excess of 100, more preferably in excess of 125 and most preferably between 125 and 500. Examples of commercial oils used as cosmetic emollient oils include mineral oil, castor oil, vegetable oil, corn oil, peanut oil, jojoba oil, 2-ethylhexyl oxystearate (and other alkyl oxystearates), acetulated lanolin alcohol, alkyl palmitates such as isopropyl palmitate, 2-ethylhexyl palmitate, glyceral triacetates, disopropyl adipate, dioctyl adipate (and other alkyl adipates), isopropyl myristate, $C_{12}$ to $C_{15}$ alcohol benzoates, and the like.

The polymeric additive must be dispersible or soluble in the oil so as to increase its viscosity. These materials are preferably polymers even though waxy substances may be used although with less desirable results. The polymers should be oleophilic to be wetted or soluble in the oil. Examples of preferred polymers include polyolefins, polystyrene, polybutadiene, graft or block polymers of these materials such as a polystyrene-polybutadiene-polystyrene block copolymer, polyacrylates, natural rubber (not heavily vulcanized), polyisoprene, polyisobutylene, cellulose acetate esters such as cellulose acetate butyrate and cellulose acetate proprionate, and the like. It has been found in the practice of the present invention that the increase in viscosity in the oil causes an increase in the average size of the microcapsules. This has not previously been reported.

The thickening additives must significantly increase the viscosity of the oils in order to have any effect on the size of the microcapsules. Viscosities at 20°C must be elevated well above 50 centipoise usually above 75 antipoise and preferably above 100 centipoise to be of significant benefit in the practice of the present invention. The thickening agents should therefore be chosed to be swellable rather than completely soluble in the oil. In U.S. Patent 3,516,941, for example, it is suggested that Piccolyte® resins be aded to cyclohexane solvents in the encapsulation process. Even at levels of 10% by weight or more of these resins viscosities are well below 50 centipoise (e.g., 10% Piccolyte 19 in cyclohexane displayed a viscosity of 5 centipoise) and are insufficient at any reasonable additive level to thicken the solvent to a degree to significantly increase the microcapsule size.

The process for making the microcapsules used in the practice of the present invention utilizes the addition of viscosity increasing materials selected from the group consisting of particulates (e.g., clays and polymeric particles), waxes, and polymeric additives to liquids and oils such as cosmetic emollient oils to increase their viscosity and then using the higher viscosity oil mixtures or solutions in a microencapsulation process to produce particles of a larger size than would ordinarily be formed in encapsulation of the cosmetic emollient oil without additives under identical encapsulation reaction conditions. Polymeric additives are especially preferred because they are more consistent and repeatable in their performance and because they hold the oil better on the skin. These oils with increased viscosity are particularly beneficial in encapsulation processes where shear forces are used to maintain a dispersed phase of oil in the reaction vessel.

The shell material of the capsule may be any of the various materials known to be useful in forming frangible and especially brittle capsules such as organic polymers, particularly phenolic-aldehydes, urea-aldehydes, acrylic polymers, addition polymers, and condensation polymers. The capsules are preferably between 50 and 2000 microns in diameter, more preferably between 100 and 1800 microns and most preferably between 200 and 1500 microns. Preferably they having a loading of (emollient and polymer)/(shell) at least 2:1 and preferably between 3:1 and 10:1.

Additional additives such as perfumes, antifungal agents, vitamins, sunscreens, and other medication may be added to the oil/polymer mixture, blended with the capsules, or used independently from the oils in the scalp cleansing, shampoo, or hair lotion composition. These additives, particularly when blended with the capsules after they have been made, may be dispersed in a creme, oil, lotion, or other media as a carrier for the capsules. In such media, the capsules would usually constitute from 2 to 100% by weight of the total cosmetic composition, preferably 25 to 80% by weight of the composition, most preferably between 40 and 80% by weight.

Skin care compositions, cleansing materials, and lotions are well known commercial products. Skin care compositions include astringents, cold cremes, sunburn treatments, antifungal ointments and creams, emollient compositions, antiviral compositions (e.g., for treatment of acne) and the like. These skin care compositions may or may not contain any cleansing or detergent materials and usually consist of blends of various ingredients such as solvents or carrying liquids (e.g., water, oils, alcohols), thickeners, emollients, surface active agents, treatment oils (skin absorbed oils), dyes, pigments, perfumes, medicines (e.g., antifungal agents, bacteriacides, antiinflamatants, etc.) and other active agents. In order to have more penetration into the scalp, skin penetrating solvents, particularly dimethylsulfoxide, may be used.

Cleansing materials or compositions are typically similarly consituted as are the skin care compositions, but contain additional compounds specifically intended to assist in the cleansing of the skin. These additional materials include detergents, oil absorbing materials, and surface active agents in concentrations sufficient to cleanse the skin. Shampoos are also included in this group, although less preferred because the baldness impending compound tends to be washed away rather than deposited on the scalp.

Lotions are also closely related to skin care compositions but tend to be used in a manner where they reside on the skin or treated area for generally longer periods of time.

Cleansing materials tend to be broken down themselves into different classes: cleansers, water removable cleansers, tissue-off cleansers, and detergent cleansers.

The generally larger, brittle particles of the present invention add unique benefits to the compositions and lotions in which they are used. Not only do the microcapsules release their contained liquid entirely or at least primarily at the time of application of the composition to the human scalp, but the brittle capsules provide a beneficial mild abrasive or defoliating effect on the surface to which it is being applied. Furthermore, this abrasive action can be readily controlled by adjusting the size and/or brittleness of the capsule shells. The shells continue to break down in size with continued physical action (e.g., rubbing, scrubbing, massaging) on the composition and lose their abrasive activity. The microcapsules can therefore be designed to provide at least a threshhold minimum of abrasion and yet put a controlled upper limit on the amount of abrasion by selecting materials that will break down to a non-abrasive size with a predetermined amount of use.

The polymeric shell of the microcapsules should be able to provide measurable abrasive activity (e.g., exfoliation or reddening from abrasion) when the microcapsules are broken into sections which average about one-eighth the diameter of the original diameter size of the microcapsules. This abrasive activity can be checked easily against the skin on the back of ones hand. Such abrasion would not be available from gelatin capsules.

The microcapsules should be stable within any carrying medium used. 'Stable' means that the microcapsules will not dissolve for a period of at least one year and that no more than fifty percent of the microcapsules should settle to the bottom of the compositions in less than six months.

## Example 1

To 900 grams of a mixture of $C_{12}$-$C_{15}$ alcohol benzoates was added 100 grams of a styrene-isoprene-styrene block copolymer (Kraton® 1107). The mixture was heated for four hours at 120°C until the copolymer had dissolved. The thickened oil was encapsulated in a urea-formaldehyde capsule according to the teachings of U.S. Patent No. 3,516,941 with the shear rate controlled to generate capsules having an average diameter between 300 and 400 microns. These capsules could be rubbed onto the skin, either directly by hand or with a brush applicator and ruptured. The oils would spread evenly on the skin and the broken capsule shells provide a useful, mildly abrasive action on the skin.

## Example 2

720 grams of a mixture of $C_{12}$-$C_{15}$ alcohol benzoates were mixed with 80 grams of the block copolymer of Example 1 and heated to 120°C with stirring until completely dissolved. The solution was cooled to 60°C and 200 grams of a c50% by weight solution of 6-piperidino-2,4-diaminopyrimidine-3-oxide dissolved in an alcohol was added to 790 grams of the solution. This mixture was cooled to 40°C and 10 grams of fragrance was added with stirring. This solution was then encapsulated according to the procedures of Example 1. The capsules could be useful as a directly applied hair restorative composition. The capsules could also be blended with a wax or cream to form a composition then could be applied to the scalp.

## Example 3

Twenty-five grams of the copolymer of Example 1 were dissolved in 975 grams of 2-ethylhexyl oxystearate. The mixture was heated to 100°C with stirring and dissolved in the manner described below.

The details of the encapsulation process are as follows:

To a one-liter baffled reactor were charged 379 gm urea-formaldehyde precondensate and 181 gm water. Vigorous mixing was applied and 80.1 gm sodium chloride and 0.53 gm sodium carboxymethyl cellulose were added. To the reactor was then added 250.8 gm of the fill material of Example 1 and precise temperature and mixing speed were applied. Sulfuric acid catalyst was added to achieve a pH of 2.5. This condition was held for two hours followed by an increase in temperature to 140°F for 2 hours. The reaction was cooled to room temperature and neutralized to a pH of 8.0. The resulting capsules were filtered, washed, and dried. The excellent quality capsules were determined to have a median size of 354 microns.

To a one liter baffled reactor were charged 303.2 gm urea-formaldehyde precondensate and 221 gm water. Vigorous mixing was applied, followed by the addition to the reactor of 37.8 gm sodium sulfate and 0.5 gm sodium carboxymethyl cellulose. After achieving solution 297 gm of the fill material, as of Example 2, was added. Precise mixing speed and temperature control were applied followed by the addition of sulfuric acid to pH 2.3. Conditions were held for three hours followed by temperature increase to 140°F for two hours. The excellent quality capsules having a median size of 61 microns were filtered, washed, and dried to a slightly clumped product.

To a 19 liter baffled reactor were added 7525 gm urea-formaldehyde precondensate and 4000 gm water. Vigorous mixing was applied followed by addition of 1650 gm sodium chloride and 11.0 gm sodium-carboxymethyl cellulose. After obtaining solution 4465 gm of the fill of Example 3 was added. Precise temperature and turbine speed controls were established, followed by addition of dilute hydrochloric acid to a pH of 2.31. This condition was held for two hours followed by a temperature increase to 140°F for 1.75 hours. The resulting capsules having a median size of 330 microns were of excellent quality.

The abrasiveness of materials with respect to skin exfoliation can be quantified by measuring the amount of fluorescence lost from skin which has been stained with a particular amount and type of fluorescent dye (in these examples, dansyl clorid was used). Fluorescence is measured before and after exfoliation with the abrasive.

These capsules were used at 5, 8, and 10% by weight levels in a detergent cream cleanser base to show the manner of performing this test. Exfoliation properties were determined in comparison to A) a cleansing cream soap, B) 5% capsules in a cream, C) 8% capsules in a cream, and D) 10% capsules in a gel cleanser and E) a commercial cleansing cream with crushed apricot pits.

A block of at least four test sites, approximately 3 cm in diameter, was mapped out on the middle back of all subjects. A previously prepared 0.1 ml aliquot of a 5% suspension of dansyl chloride in a white petrolatum base was then applied to each test site using an occlusive dressing. After a twenty-four (24) hour exposure, the patches were removed and the areas gently washed and blotted dry with a soft terry cloth towel to remove unincorporated dye. The sites were then allowed an additional twenty-four (24) hour air exposed resting period before application of test products.

On day three of the study, the subjects returned to the test laboratory to have the test sites photographed under carefully controlled conditions including UV illumination. Approximately 0.25 gm of each test product was applied to the test sites according to a predetermined randomization schedule.

Control and treatment sites were cleansed for thirty (30) seconds using a rapid circular motion, medium hand pressure and tepid water. Fingers were used to cleanse both the treatment and control sites. Lather from a cream cleansing bar were used on the control sites, and the compositions were then used to cleanse the treatment sites. After cleansing, test sites were rinsed with tepid water, patted dry, and again photographed under UV light. This procedure of treatment, then photographing the treated sites was repeated an additional three times, or until all test products could be visually differentiated from the control site.

To assure uniformity of photographs, all film used in this study was from the same bulk roll of film, and was submitted for special batch processing. Uniformity of photographs is important as they were examined by a Nikon Magiscan 2A Image Analysis system for a quantitative determination of the percent decrease in florescence as compared to control. The greater the decrease in florescence, the greater the exfoliating capacity of the treatment.

The commercial cream cleansing bar (A) provided essentially 0% reduction in fluorescence as compared to a water wash. The apricot abrasive (E) produced a 12.4% further reduction in fluoresence, and the compositions (B, C, D) of claims 16, 17, and 18 provided a reduction in fluorescence of 2.5%, 4% and 6%.

It is thus preferred to have an amount of brittle microcapsules present in the material to be applied to the skin which can reduce fluorescence by at least 1%, preferably at least 2%, and most preferably at least 2.5%, but preferably not greater than 10% (to prevent overscrubbing) than is reduced by tepid water washing under equivalent applied pressure to the skin area.

The 2,4-pyrimidine diamine, 6-(1-piperidinyl oxide was first dissolved in ethanol and added to the compositions encapsulated with the additional materials of Example 3 to provide a 10% loading of the restorative. These capsules were then incorporated into hair lotion at 50% by volume loading. The lotion was virorously rubbed into the scalp, breaking the capsules, exfoliating the scalp, and applying a hair restorative effective amount of the restorative to the scalp.

"Mild abrasion" or a "mild abrasive" is a material which when present in a shampoo carrying medium increases the amount of fluorescent dye removed in the above test by 1-25% (preferably 1-20% and most preferably at least 2-15%) as compared to a tepid (25°C) water wash under equivalent pressure (as manually applied).

## Claims

1. Compositions which can be applied to the scalp comprising brittle microcapsules of encapsulated 6-piperidino-2,4-diaminopyrimidine-3-oxide, said microcapsules having average diameters between 50 and 2500 microns.

2. The compositions of claim 1 having average diameters between 100 and 2000 and where said 6-piperidino-2,4-diaminopyrimidine-3-oxide is dissolved in a solvent.

3. The compositions of claims 1-2 wherein a thickener and an oil is present in said microcapsules and said oil is an emollient oil and the oil plus thickener has a viscosity between 300 and 1500 cp at 20° C.

4. The compositions of claim 3 wherein said oil is an emollient oil and is selected from the group consisting of mineral oil, castor oil, jojoba oil, vegetable oil, 2-ethylhexyl oxystearate, $C_{12}$-$C_{15}$ alcohol benzoates, isopropyl palmitate and isopropyl myristate.

5. The compositions of claim 3 wherein said polymeric thickening agent is selected from the group consisting of polyolefins, polybutadiene, polystyrene, polyacrylics, gelatin, natural rubber, polyisoprene, cellulose acetate esters and copolymers thereof.

6. The compositions of claim 4 wherein said polymeric thickening agent is selected from the group consisting of polyolefins, polybutadiene, polystyrene, polyacrylics, gelatin, natural rubber, polyisoprene, cellulose acetate esters and copolymers thereof.

7. The composition of claim 3 further comprising as a carrying medium for said microcapsules at least three ingredients selected from the group consisting of oils, alcohols, surface active agents, detergents, dyes, thickening agents and aqueous mediums.

8. A composition for application to the scalp comprising a carrying medium comprising a liquid or a cream, said medium being characterized by containing both at least 20% by weight of a hair-loss preventative compound and at least 2% by weight of a mild abrasive.

9. The composition of claim 8 wherein a skin penetrating solvent is present in said composition.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 177 223 (M. MEZEI) <br> * whole document * <br> --- | 1-9 | A 61 K 7/06 <br> A 61 K 9/50 <br> B 01 J 13/00 |
| A | EP-A-0 211 268 (V. ZAPPIA) <br> * claims * <br> --- | 1-9 | |
| A | EP-A-0 133 295 (MITSUI TOATSU CHEMICALS INC.) <br> * claims; page 20, lines 8-27 * <br> --- | 1-9 | |
| A | ULLMANN'S ENCYCLOPEDIA OF INDUSTRIAL CHEMISTRY <br> vol. A 9, 5th edition, pages 297-339, VCH Verlagsgesellschaft mbH, Weinheim, DE; "Emulsions" * pages 318-320 * <br> ----- | | |

TECHNICAL FIELDS SEARCHED (Int. Cl.4)

A 61 K
B 01 J

The present search report has been drawn up for all claims

| Place of search | Date of completion of the search | Examiner |
|---|---|---|
| BERLIN | 19-05-1989 | SIATOU E |